# EUROPEAN PATENT APPLICATION

(11) **EP 0 524 677 A1**
(43) Date of publication of application: **27.01.1993**
(21) Application number: 92202081.3
(22) Date of filing: 08.07.1992
(51) Int. Cl.: C07D 493/08, C07H 19/01, A61K 31/34

(54) **Cyclic ketal derivatives**

(30) Priority: 16.07.1991 GB 9115247; 06.11.1991 GB 9123575
(71) Applicant: GLAXO GROUP LIMITED, Greenford, Middlesex UB6 0NN (GB)
(72) Inventor: Sidebottom, Philip James Glaxo Group Research Ltd., Greenford,Middlesex, UB6 0HE (GB); Watson, Nigel Stephen Glaxo Group Research Ltd., Greenford, Middlesex, UB6 0HE (GB); Ross, Barry Clive Glaxo Group Research Ltd., Ware, Hertfordshire, 12 0DG (GB); Procopiou, Panayotis Alexandrou, Greenford, Middlesex UB6 0HE (GB); Lester, Michael George, Greenford, Middlesex UB6 0HE (GB); Chan, Chuen, Greenford, Middlesex UB6 0HE (GB); Andreotti, Daniele, I-37100 Verona (IT)
(74) Representative: Caffin, Lee

(57) **Abstract**

Compounds are described of the formula 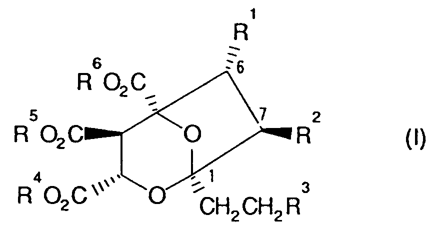
(wherein R¹ represents a hydroxyl group or a group selected from -OCOCH 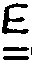 CHCH(CH₃)(CH₂)₃CH₃,-OCOCH 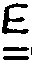 CHC(CH₃) 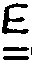 CHCH(CH₃)CH₂CH₃ or -OCO-X-CH₂CH(CH₃)CH₂CH₃ [where X is -CH 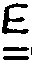 CHCH(CH₃)-, -CH₂CH(OH)CH(CH₃)-, - CH 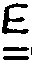 CHC(OH)(CH₃)-, -CH₂CH (OH)CH₂- or-CH₂CH₂CH(CH₃)-];
R² represents a hydroxyl group or an acyloxy group selected from formyloxy and acetoxy;
R³ represents a group selected from 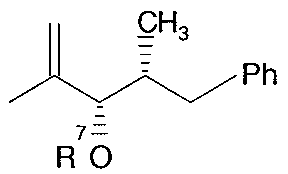
(where R⁷is a hydrogen atom or an acetyl group), -(CH₃) 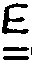 CHCH(CH₂R⁸)CH₂Ph (where R⁸ is a hydrogen or a hydroxyl group),-C(CH₂OH) 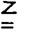 CHCH(CH₃)CH₂Ph, -C(=CH₂)CH(OH)CH (CH₂OH)CH₂Ph,-C(=CH₂)CH(NHCOCH₃)CH(CH₃)CH₂Ph, C(CH₂NHCOCH₃) 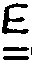 CHCH(CH₃)CH₂Ph and 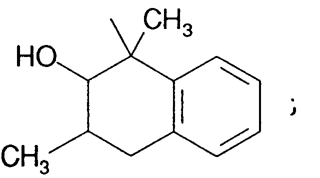
R⁴, R⁵ and R⁶ may each independently represent a hydrogen atom or a methyl group; and salts thereof.

These compounds inhibit the enzyme squalene synthase and/or are intermediates for the preparation of compounds which inhibit the enzyme squalene synthase. Compounds of the inventions may be formulated for use in a variety of conditions where a lowering of the level of blood plasma cholesterol in animals would be beneficial and for use in combating fungal infections in animals.

## Description

This invention relates to novel compounds having hypocholesterolemic, hypolipidemic and/or antifungal activity, to processes for their preparation, to pharmaceutical compositions containing them and to their use in medicine, particularly in the treatment and/or prevention of atherosclerosis and associated cardiovascular diseases. The invention also relates to novel compounds which are useful as intermediates for the preparation of compounds having hypocholesterolemic, hypolipidemic and/or antifungal activity.

High levels of blood cholesterol and blood lipids are conditions which are implicated in the onset of vessel wall disease. Methods for effective reduction of plasma cholesterol levels are therefore of high interest. Cholesterol concentrations can be reduced, for example, by lowering the dietary intake of the sterol, by enhancing its metabolism and elimination or by decreasing its rate of biosynthesis. The most effective approaches to lowering physiological cholesterol levels are likely to include inhibition of cholesterol biosynthesis as a component since cholesterol synthesis is subject to feedback regulation, so that decreases in cholesterol levels tend to be compensated for by increased biosynthesis.

One rate-controlling step in the biosynthesis of cholesterol is the formation of mevalonic acid from 3-hydroxy-3-methylglutaryl coenzyme A (HMG CoA) and clinical successes have been achieved with the mevinic acid family of HMG CoA reductase inhibitors in the treatment of hypercholesterolemia. Mevalonic acid, however, is a common precursor of all isoprenyl derivatives, including in animals coenzyme Q, heme A and the dolichols.

The first biosynthetic step which leads exclusively to sterols, the condensation of two farnesyl pyrophosphates to give squalene, is a second site of regulation. The synthesis of squalene from farnesyl pyrophosphate involves an isolable intermediate, presqualene pyrophosphate, and the entire synthetic sequence is catalysed by squalene synthase (farnesyldiphosphate: farnesyldiphosphate farnesyltransferase, EC 2.5.1.21), a membrane -bound enzyme. Agents which act to inhibit the enzyme squalene synthase are therefore potential drugs for the regulation of cholesterogenesis. The use of such agents is attractive as non- steroidal pathways should be minimally affected.

The biosynthesis of ergosterol, the major sterol component of fungal cell membranes, is analogous to that of cholesterol in mammals, including humans, and is thus mediated by the enzyme squalene synthase. Agents which act to inhibit the enzyme squalene synthase in fungal cells are therefore potential drugs for antifungal chemotherapy.

We have now found a group of novel compounds which act as inhibitors of the enzyme squalene synthase and/or are intermediates for the preparation of compounds which act as inhibitors of the enzyme squalene synthase.

Thus, in a first aspect of the present invention, we provide compounds of the general formula (I) 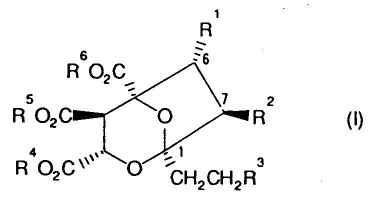
[wherein R¹ represents a hydroxyl group or a group selected from -OCOCH 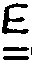 CHCH(CH₃)(CH₂)₃CH₃,-OCOCH 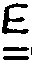 CHC(CH₃) 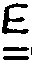 CHCH(CH₃)CH₂CH₃ or -OCO-X-CH₂CH(CH₃)CH₂CH₃ [where X is -CH 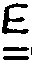 CHCH(CH₃)-, -CH₂CH(OH)CH(CH₃)-, - CH 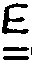 CHC(OH)(CH₃)-, -CH₂CH (OH)CH₂- or -CH₂CH₂CH(CH₃)-];
R² represents a hydroxyl group or an acyloxy group selected from formyloxy and acetoxy;
R³ represents a group selected from 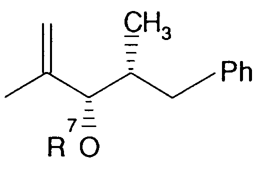
(where R⁷ is a hydrogen atom or an acetyl group),-(CH₃) 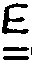 CHCH(CH₂R⁸)CH₂Ph (where R⁸ is a hydrogen or a hydroxyl group),-C(CH₂OH) 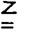 CHCH(CH₃)CH₂Ph, -C(=CH₂)CH(OH)CH(CH₂OH)CH₂Ph,-C(=CH₂)CH(NHCOCH₃)CH(CH₃)CH₂Ph, -C(CH₂NHCOCH₃) 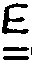 CHCH(CH₃)CH₂Ph and 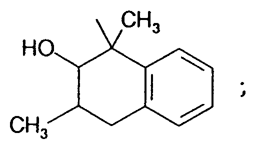
R⁴, R⁵ and R⁶ may each independently represent a hydrogen atom or a methyl group; and salts thereof.

Compounds of formula (I) in which R⁴, R⁵ and R⁶ represent hydrogen atoms or physiologically acceptable cations are generally preferred.

R¹ preferably represents a group 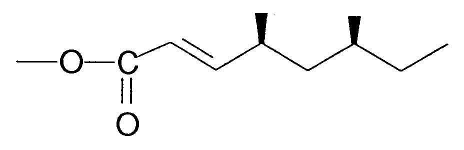
R² preferably represents a hydroxyl group.

R³ preferably represents a group 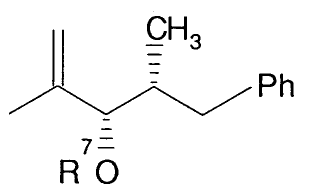
(where R⁷ is a hydrogen atom or an acetyl group).

It is to be understood that this invention covers any combination of the abovementioned preferences.

A particularly preferred compound of formula (I) is [1S- [1α(4R*,5S*),3α,4β,5α,6 α(2E,4R*,6R*),7β]] 1-(4-acetyloxy-5-methyl-3- methylene-6-phenylhexyl)-6 ,7-dihydroxy -2,8-dioxabicyclo [3.2.1] octane-3,4,5-tricarboxylic acid, 6-(4,6-dimethyl-2-octenoate) and physiologically acceptable salts thereof.

Compounds of formula (I) in which R¹ represents a hydroxyl group are particularly useful as intermediates for the preparation of related structures having squalene synthase inhibitory activity.

Compounds of the present invention may form salts with inorganic and organic bases. Physiologically acceptable salts include inorganic base salts such as alkali metal salts (e.g. sodium and potassium salts including the trisodium, dipotassium and tripotassium salts), alkaline earth metal salts (e.g. calcium salts) and ammonium salts Suitable organic base salts include amine salts such as trialkylamine (e.g. triethylamine), dialkylamine (e.g. dicyclohexylamine), optionally substituted benzylamine (e.g. p-bromobenzylamine), tris(hydroxymethyl)methylamine salts and amino acid salts (e.g. lysine and arginine salts including the tri-L-lysine salts).

Compounds of the invention have been found to inhibit the enzyme squalene synthase and cholesterol biosynthesis and are therefore of use in medicine, particularly in a variety of conditions where a lowering of the level of blood plasma cholesterol in animals (especially humans) would be beneficial. Examples of such conditions include diseases associated with hypercholesterolemia and hyperlipoproteinemia, especially atherosclerosis and cardiovascular diseases (such as cardiac ischaemic diseases, cerebral ischaemic diseases and peripheral arterial diseases).

Compounds of the invention which inhibit squalene synthase may also be of use in combating fungal infections in animals, including humans. For example, they may be useful in the treatment of systemic infections caused by, for example Candida (e.g. Candida albicans, Candida glabrata, Candida parapsilosis and Candida pseudotrop), Cryptococcus neoformans, Aspergillus Sp (e.g. Aspergillus flavus and Aspergillus fumigatus), Coccidioides (e.g. Coccidioides immitis), Paracoccidioides (e.g. Paracoccidioides brasiliensis), Histoplasma (e.g. Histoplasma capsulatum) or Blastomyces (e.g. Blastomyces dermatitidis). They may also be useful in treating topical infections caused by species of Trichophyton, Microspotum or Epidermophyton (e.g. Trichophyton mentographytes, Microsporum canis or Epidermophyton floccosum). They may also be of use in treating fungal diseases caused by Torulopsis glabrata and Pityrosporum ovale.

The in vitro evaluation of the anti-fungal activity of compounds of the invention can be performed by determining the minimum inhibitory concentration (MIC) which is the concentration of the test compound in a suitable medium at which growth of a particular microorganism fails to occur.

In view of their potential in antifungal therapy, compounds of the invention which inhibit squalene synthase may recommend themselves for the treatment of a variety of fungal infections in human beings and animals. Such infections include mycotic infections such as candidiasis and chronic mucocandidiasis (e.g. thrush and vaginal candidiasis) and skin infections caused by fungi, cutaneous and mucocutaneous candidiasis, dermatophytoses including ringworm and tinea infections, athletes foot, paronychia, pityriasis versicolor, erythrasma, intertrigo, fungal nappy rash, candida vulvitis, candida balanitis and otitis externa. They may also be useful as prophylactic agents to prevent systemic and topical fungal infections. Use as prophylactic agents may, for example, be appropriate as part of a selective gut decontamination regimen in the prevention of infection in immunocompromised patients. Prevention of fungal overgrowth during antibiotic treatment may also be desirable in some disease syndromes or iatrogenic states.

The ability of compounds of the invention to inhibit the enzyme squalene synthase in mammals and fungi may be demonstrated in vitro using [2-¹⁴C]farnesylpyrophosphate as a substrate under assay conditions similar to those described by S. A. Biller et al. in J. Medicinal Chemistry 31(10), 1869-1871 (1988); [¹⁴C]squalene is separated from unreacted substrate on thin layer chromatography plates and determined by liquid scintillation counting. The ability of compounds of the invention to inhibit cholesterol biosynthesis may be demonstrated by measuring inhibition from [¹⁴C]-acetate in liver slices from male Wistar rats using a method similar to that described by Y. Tsujita et al. in Biochem. Biophys. Acta, Volume 877, 50-60 (1986) and modified to include measurement of cholesterol by high performance liquid chromatography (h.p.l.c.).

While it is possible that, for use in therapy, compounds of the invention which inhibit squalene synthase may be administered as the raw chemical, it is preferable to present the active ingredient as a pharmaceutical formulation. The invention thus further provides a pharmaceutical formulation comprising compounds of the invention which inhibits squalene synthase together with one or more pharmaceutically acceptable carriers thereof and, optionally, other therapeutic and/or prophylactic ingredients. The carrier(s) must be 'acceptable' in the sense of being compatible with the other ingredients of the formulation and not deleterious to the recipient thereof.

The compositions of the invention include those in a form especially formulated for oral, buccal, parenteral, implant, rectal, topical, ophthalmic or genito-urinary administration or in a form suitable for administration by inhalation or insufflation.

Tablets and capsules for oral administration may contain conventional excipients such as binding agents, for example, syrup, acacia, gelatin, sorbitol, tragacanth, mucilage of starch or polyvinylpyrrolidone; fillers, for example, lactose, sugar, microcrystalline cellulose, maize-starch, calcium phosphate or sorbitol; lubricants, for example, magnesium stearate, stearic acid, talc, polyethylene glycol or silica; disintegrants, for example, potato starch or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup, gelatin, hydroxyethylcellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example, almond oil, fractionated coconut oil, oily esters, propylene glycol or ethyl alcohol; and preservatives, for example, methyl or propyl p-hydroxybenzoates or sorbic acid. The compositions may also be formulated as suppositories, e.g. containing conventional suppository bases such as cocoa butter or other glycerides.

For buccal administration the composition may take the form of tablets or lozenges formulated in conventional manner.

The composition according to the invention may be formulated for parenteral administration by injection or continuous infusion. Formulations for injection may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

For administration by inhalation the compositions according to the invention are conveniently delivered in the form of an aerosol spray presentation from pressurised packs with the use of a suitable propellant, e.g. dichlorodifluoromethane, trichlorofluoromethane, dichlorotetrafluoroethane, carbon dioxide or other suitable gas, or from a nebuliser. In the case of a pressurised aerosol the dosage unit may be determined by providing a valve to deliver a metered amount.

Alternatively, for administration by inhalation the compositions according to the invention may take the form of a dry powder composition, for example a powder mix of the compound and a suitable powder base such as lactose or starch. The powder composition may be presented in unit dosage form in, for example, capsules or cartridges of e.g. gelatin, or blister packs from which the powder may be administered with the aid of an inhaler or insufflator.

The compositions may take the form of a suppository, e.g. containing a conventional suppository base, or a pessary, e.g. containing a conventional pessary base.

The compositions may also be formulated for topical administration in the form of ointments, creams, gels, lotions, shampoos, powders (including spray powders), pessaries, tampons, sprays, dips, aerosols, drops (e.g. eye, ear or nose drops) or pour-ons. Ointments and creams may, for example, be formulated with an aqueous or oily base with the addition of suitable thickening and/or gelling agents. Ointments for administration to the eye may be manufactured in a sterile manner using sterilised components. Pour-ons may, for example, be formulated for veterinary use in oils containing organic solvents, optionally with formulatory agents, e.g. stabilising and solubilising agents. Pessaries and tampons for vaginal insertion may be formulated using conventional techniques and, where appropriate, may contain an effervescent vehicle. Such compositions may also contain other active ingredients such as corticosteroids, antibiotics or antiparasitics as appropiate.

Liquid preparations for intranasal delivery may take the form of solutions or suspensions and may contain conventional excipients such as tonicity adjusting agents, for example, sodium chloride, dextrose or mannitol; preservatives, for example benzalkonium chloride, thiomersal, phenylethyl alcohol; and other formulating agents such as suspending, buffering, stabilising and/or dispersing agents.

Transdermal administration may be affected by the design of a suitable system which promotes adsorption of the active compound through the skin and would typically consist of a base formulation enclosed within an adhesive stick-on patch comprising backing films, membranes and release liners.

The composition according to the invention may also be formulated as a depot preparation. Such long acting formulations may be administered by implantation (for example subcutaneously or intramuscularly) or by intramuscular injection. Thus, for example, a compound of the invention may be formulated with suitable polymeric or hydrophobic materials (for example as an emulsion in an acceptable oil) or ion exchange resins, or as sparingly soluble derivatives, for example, as a sparingly soluble salt.

When the compositions comprise dosage units, each unit will preferably contain 0.001mg to 1000mg, advantageously 0.01mg to 400mg, of active ingredient where a compound of the invention is to be administered orally. The daily dosage as employed for adult human treatment will preferably range from 0.001mg to 5000mg of active ingredient, most preferably from 0.01mg to 2000mg which may be administered in 1 to 4 daily doses, for example, depending on the route of administration and on the condition of the patient and the disease to be treated.

The compound may be administered by intravenous infusion using, for example, up to 50mg/kg/day of the active ingredient. The duration of treatment will be dictated by the rate of response rather than by arbitrary numbers of days.

Compounds of the invention which inhibit squalene synthase may also be used in combination with other therapeutic agents, and the invention thus provides, in a further aspect, a combination comprising a compound of the invention which inhibits squalene synthase together with another therapeutically active agent, such as an inhibitor of 3-hydroxy-3-methylglutaryl coenzyme A (HMG CoA) reductase or another agent which reduces serum cholesterol and/or inhibits cholesterol biosynthesis, for example a bile acid sequestrant or an antihyperlipoproteinemic or antihyperlipemic agent such as probucol, gemfibrozil, clofibrate, dextrothyroxine or its sodium salt, colestipol or its hydrochloride salt, cholestyramine, nicotinic acid, neomycin, p-aminosalicylic acid, aspirin, DEAE-Sephadex, a poly(diallylmethylamine) derivative, an ionene or poly(diallyldimethylammonium) chloride.

The combinations referred to above may conveniently be presented for use in the form of a pharmaceutical formulation and thus pharmaceutical formulations comprising a combination as defined above together with a pharmaceutically acceptable carrier thereof comprise a further aspect of the invention. The individual components of such combinations may be administered either sequentially or simultaneously in separate or combined pharmaceutical formulations.

When a compound of the invention is used in combination with a second therapeutic agent against the same condition the dose of each compound may differ from that when the compound is used alone. Appropriate doses will be readily appreciated by those skilled in the art.

According to another aspect of the present invention, we provide a compound of formula (I) or a physiologically acceptable salt thereof or a pharmaceutical composition comprising a compound of formula (I) or a physiologically acceptable salt thereof as defined above for use in therapy, particularly for the treatment of conditions where a lowering of the level of blood plasma cholesterol in animals (especially humans) would be beneficial, or for the treatment of fungal infections in animals (especially humans).

In a particular aspect of the present invention, we provide a compound of formula (I) or a physiologically acceptable salt thereof or a pharmaceutical composition comprising a compound of formula (I) or a physiologically acceptable salt thereof as defined above for use in the treatment of diseases associated with hypercholesterolemia and/or hyperlipoproteinemia, especially atherosclerosis and cardiovascular diseases (such as cardiac ischaemic diseases, cerebral ischaemic diseases and peripheral arterial disease).

According to a further aspect of the present invention, we provide the use of a compound of formula (I) or a physiologically acceptable salt thereof in the manufacture of a medicament for the treatment of diseases associated with hypercholesterolemia and/or hyperlipoproteinemia, especially atherosclerosis and cardiovascular diseases (such as cardiac ischaemic diseases, cerebral ischaemic diseases and peripheral arterial disease).

According to another aspect of the present invention, we provide the use of a compound of formula (I) or a physiologically acceptable salt thereof in the manufacture of a medicament for the treatment of fungal infections in a human or non-human animal patient.

According to a further aspect of the present invention, we provide a method of treatment of the human or non-human animal body to combat diseases associated with hypercholesterolemia and/or hyperlipoproteinemia, especially atherosclerosis and cardiovascular diseases (such as cardiac ischaemic diseases, cerebral ischaemic diseases and peripheral arterial disease or to combat fungal diseases, which method comprises administering to said body an effective amount of a compound of formula (I) or a physiologically acceptable salt thereof.

It will be appreciated by those skilled in the art that references herein to treatment extend to prophylaxis as well as the treatment of established conditions or infections.

The compounds of the invention may be prepared by the processes described below.

Thus, a process (A) for the preparation of compounds of formula (I) in which R² is a hydroxyl group or an acetoxy group comprises reacting a compound of formula (II) 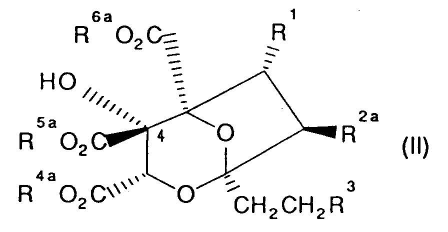
(wherein R¹ and R³ are as defined previously, R^{2a} is an acetoxy group or a protected hydroxyl group and R^{4a}, R^{5a} and R^{6a} are protecting groups) to replace the 4-hydroxyl group with a hydrogen atom, followed by removal of the protecting groups present.

The reaction to remove the 4-position hydroxyl group may conveniently be effected by activation of the 4-hydroxyl group, followed by reduction with a suitable reducing agent such as a silane (e.g. tris(trimethylsilyl)silane) or an alkyltin hydride (e.g. tributyltin hydride) in the presence of a radical initiator (e.g. azobisisobutyronitrile) and conveniently at an elevated temperature (eg 50-120°C) in a solvent such as an aromatic hydrocarbon (eg toluene or xylene).

Activation of the 4-hydroxyl group may be effected, for example, by conversion to a compound of formula (III) 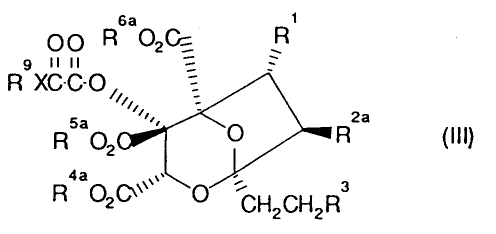
(wherein R¹, R^{2a}, R³, R^{4a}, R^{5a} and R^{6a} are as defined previously, R⁹ is a C₁₋₆alkyl group and X is oxygen or sulphur). Compounds of formula (III) in which X is an oxygen atom may be prepared by treating a compound of formula (II) with an alkyl oxalyl halide (e.g. methyl oxalyl chloride) in the presence of a base such as an organic base (e.g. triethylamine or dimethylaminopyridine or a mixture of such bases) or an inorganic base (eg sodium hydride). Compounds of formula (III) in which X is a sulphur atom may be prepared by treating a compound of formula (II) with oxalyl chloride in the presence of a suitable base as defined just above followed by treatment with an alkyl mercaptan (e.g. t- butyl mercaptan) in the presence of a suitable base as defined just above. If desired, compounds of formula (II) may be converted to compounds of formula (I) without isolating intermediate compounds of formula (III).

Compounds of formula (I) may be separated from the corresponding 4-α-CO₂R⁵ epimers by conventional chromatographic means.

Compounds of formula (II) may conveniently be prepared from compounds of formula (IV) 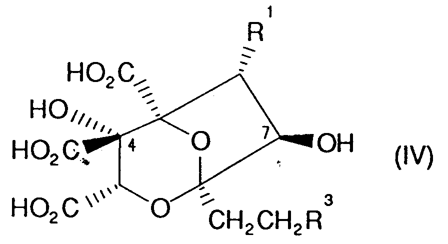
(wherein R¹ and R³ are as defined in formula (I) above) by standard selective protection techniques, with acetylation, if appropriate, at the 7-position.

Suitable carboxylic acid protecting groups and hydroxyl protecting groups include any conventional protecting group, for example as described in 'Protective Groups in Organic Chemistry', Ed. J. F. W. McOmie(Plenum Press, 1973) or 'Protective Groups in Organic Synthesis' by Theodora W. Greene (John Wiley and Sons, 1981). Examples of suitable carboxylic acid protecting groups include alkyl groups such as methyl or t-butyl, 2-methoxyethoxymethyl or aralkyl groups such as benzyl, diphenylmethyl or p-nitrobenzyl. Examples of suitable hydroxyl protecting groups include groups such as 2-methoxyethoxymethyl, tetrahydropyran-2-yl and silyl groups such as trialkylsilyl groups (e.g. t-butyldimethylsilyl).

The protecting groups may be removed using conventional techniques. Thus, an alkyl group such as t-butyl may, for example, be removed under anhydrous acid conditions (for example using hydrogen chloride in a solvent such as an ether, eg dioxan or using formic acid). Alternatively, the removal of a methyl protecting group may be effected using lithium iodide in aqueous dimethylsulphoxide or 2,4,6-trimethylpyridine at an elevated temperature. An aralkyl group may conveniently be removed by catalytic hydrogenation using for example a suitable metal catalyst such as palladium-on-carbon. Alternatively, a p-nitrobenzyl group may conveniently be removed using zinc metal and hydrochloric acid in a solvent such as an ether (e.g. tetrahydrofuran or aqueous tetrahydrofuran). A diphenylmethyl group or a 2-methoxyethoxymethyl group may conveniently be removed using aqueous formic acid or trifluoroacetic acid. Silyl groups such as t-butyldimethylsilyl may conveniently be removed by treatment with a tetraalkylammonium halide, e.g. tetra-n-butylammonium fluoride, in an ether solvent (e.g. tetrahydrofuran). A tetrahydropyran-2-yl group may conveniently be removed by treatment with pyridinium p-toluenesulphonate.

Acetylation of a free hydroxyl group at the 7 position may be effected by treatment with acetic anhydride in a solvent such as a halogenated hydrocarbon (e.g. trichloromethane) in the presence of a base such as triethylamine or dimethylaminopyridine or a mixture thereof.

Esterification of carboxylic acids of formula (IV) to the corresponding methyl esters may conveniently be effected by treatment with a methylating agent such as a methyl halide (e.g. methyl iodide) or dimethyl sulphate in a suitable organic solvent such as an amide (e.g. dimethylacetamide or preferably dimethylformamide) in the presence of a base such as a bicarbonate (e.g. sodium bicarbonate). The reaction may conveniently be carried out at a temperature ranging from 0⁰ to 100⁰C, preferably 20⁰ to 30⁰C. Alternatively, the esterification may be effected by treatment with an ethereal solution of diazomethane in a suitable solvent such as methanol. The esterification may also be effected by treatment with methanol in the presence of a suitable acid such as a mineral acid (e.g. hydrochloric acid) at about room temperature.

Conversion of one methyl ester of formula (II) to a different methyl ester may be carried out by appropriate esterification/deesterification steps. The deesterification may be effected under standard conditions, for example by base hydrolysis.

Another process (B) for the preparation of compounds of formula (I) comprises converting a compound of formula (I) to a different compound of formula (I). In one embodiment, a compound of formula (I) in which R¹ represents a hydroxyl group may be prepared by deacylation of a corresponding compound of formula (I) in which R¹ represents an acyloxy group as defined in formula (I) above using the general deacylation conditions described hereinafter. In a further embodiment, a compound of formula (I) in which R⁷ represents hydrogen may be prepared by deacetylation of a corresponding compound of formula (I) in which R¹ represents an acetyl group using conventional means. For example, the deacetylation may be effected under acid conditions (eg using an inorganic acid such as sulphuric acid). In another embodiment, a compound of formula (I) in which R² represents a formyloxy group may be prepared by treating a corresponding compound of formula (I) in which R² represents a hydroxyl group with formic acid, conveniently at about room temperature. It will be appreciated that under such conditions compounds of formula (I) in which R² is formyloxy and R⁴-R⁶ are hydrogen may be prepared directly from compounds of formula (II) in which R^{2a} is a hydroxyl group and R^{4a}-R^{6a} are t-butyl.

Compounds of formula (IV) may be prepared according to the fermentation process described hereinafter or may be prepared from products of the fermentation process by acylation or deacylation at the 6-position as appropriate according to suitable acylation and deacylation methods. Suitable acylation methods are described hereinafter. Deacylation may conveniently be effected by base- catalysed hydrolysis using a base such as aqueous sodium hydroxide in a solvent such as an alcohol (e.g. methanol). Alternatively, deacylation of α, β unsaturated esters may be carried out using a hydroxylamine (e.g. N-methylhydroxylamine hydrochloride) optionally in the presence of a suitable base (e.g. a trialkylamine such as triethylamine) in a solvent such as dimethylformamide. The fermentation process comprises cultivating a microorganism capable of producing one or more of the appropriate compounds of formula (IV). Thereafter the desired compound from the culture may be isolated and, if desired, acylated or deacylating and/or esterified to the corresponding methyl ester.

Suitable microorganisms may readily be identified by using a small scale test and analysing a test sample obtained from fermentation of the microorganism using standard methodology.

In particular the microorganism to be conventionally used is a strain of microorganism deposited in the permanent culture collection of the CAB International Mycological Institute, Ferry Road, Kew, Surrey, England. The strain was received by the Institute on 25th May 1989 and was subsequently given the accession no. IMI 332962 and a deposit date of 27th June 1989 (date of confirmation of viability). The deposited strain is identified herein by reference to the Institute accession no. IMI 332962. The characteristics thus far identified for IMI 332962 are given in Example 10 hereinafter.

It will be appreciated that the desired intermediates may also be prepared from a mutant of IMI 332962.

Mutants of the IMI 332962 may arise spontaneously or may be produced by a variety of methods including those outlined in Techniques for the Development of Micro-organisms by H. I. Adler in 'Radiation and Radioisotopes for Industrial Microorganisms', Proceedings of the Symposium, Vienna 1973, p241, International Atomic Energy Authority. Such methods include ionising radiation, chemical methods e.g. treatment with N-methyl-N'-nitro-N- nitrosoguanidine (NTG), heat, genetic techniques, such as recombination and transformation, and selective techniques for spontaneous mutants.

The fermentation may be effected by conventional means i.e. by culturing the organism in the presence of assimilable sources of carbon, nitrogen and mineral salts.

Assimilable sources of carbon, nitrogen and minerals may be provided by either simple or complex nutrients. Sources of carbon will generally include glucose, maltose, starch, glycerol, molasses, dextrin, lactose, sucrose, fructose, galactose, myo-inositol, D-mannitol, soya bean oil, carboxylic acids, amino acids, glycerides, alcohols, alkanes and vegetable oils. Sources of carbon will generally comprise from 0.5 to 10% by weight of the fermentation medium. Fructose, glucose and sucrose represent preferred sources of carbon.

Sources of nitrogen will generally include soya bean meal, corn steep liquors, distillers solubles, yeast extracts, cottonseed meal, peptones, ground nut meal, malt extract, molasses, casein, amino acid mixtures, ammonia (gas or solution), ammonium salts or nitrates. Urea and other amides may also be used. Sources of nitrogen will generally comprise from 0.1 to 10% by weight of the fermentation medium.

Nutrient mineral salts which may be incorporated into the culture medium include the generally used salts capable of yielding sodium potassium, ammonium, iron, magnesium, zinc, nickel, cobalt, manganese, vanadium, chromium, calcium, copper, molybdenum, boron, phosphate, sulphate, chloride and carbonate ions.

Cultivation of the organism will generally be effected at a temperature of from 20 to 40⁰C preferably from 20 to 35⁰C, especially around 25 to 28⁰C, and will desirably take place with aeration and agitation e.g. by shaking or stirring. The medium may initially be inoculated with a small quantity of mycelium and/or spores. The vegetative inoculum obtained may be transferred to the fermentation medium, or to one or more seed stages where further growth takes place before transfer to the principal fermentation medium. The fermentation will generally be carried out in the pH range 3.5 to 9.5, preferably 4.5 to 7.5. It may be necessary to add a base or an acid to the fermentation medium to keep the pH within the desired range. Suitable bases which may be added include alkali metal hydroxides such as aqueous sodium hydroxide or potassium hydroxide. Suitable acids include mineral acids such as hydrochloric, sulphuric or phosphoric acid.

The fermentation may be carried out for a period of 4-30 days, preferably about 7-18 days. An antifoam may be present to control excessive foaming and added at intervals as required. Carbon and/or nitrogen sources may also be fed into the fermentation medium as required.

The products of the fermentation process may be present in both the fermentation liquor and the mycelial fraction, which may conveniently be separated by filtration or centrifugation. The liquor may be optionally thereafter treated with an acid such as sulphuric acid in the presence of an organic solvent until the pH is below, pH 6 (e.g. about pH 3).

The products of the fermentation process may be separated from the fermentation broth by conventional isolation and separation techniques. It will be appreciated that the choice of isolation techniques may be varied widely.

The products of the fermentation process may be isolated and purified by a variety of fractionation techniques, for example adsorption-elution, precipitation, fractional crystallisation, solvent extraction and liquid-liquid partition which may be combined in various ways.

Adsorption onto a solid support followed by elution has been found to be suitable for isolating and purifying compounds of the invention.

The products of the fermentation process may be extracted from the cells and the aqueous phase with an appropriate organic solvent such as a ketone (e.g. acetone, methyl ethyl ketone or methyl isobutyl ketone), a halogenated hydrocarbon, an alcohol, a diol (e.g. propane-1,2-diol or butane-1,3-diol) or an ester (e.g. methyl acetate or ethyl acetate). Generally, more than one extraction may be desirable to achieve optimum recovery. The water-immiscible solvent extracts may themselves be extracted with basic aqueous solutions, and after acidification of these basic solutions the desired compounds may be reextracted into water-immiscible organic phase. Removal of the solvent from the organic extracts (e.g. by evaporation) yields a material containing the desired compounds.

Chromatography (including high performance liquid chromatography) may be effected on a suitable support such as silica; a non-functional macroreticular adsorption resin for example cross-linked styrene divinyl benzene polymer resins such as Amberlite XAD-2, XAD-4, XAD-16 or XAD-1180 resins (Rohm & Haas Ltd) or Kastell S112 (Montedison); a substituted styrene-divinyl benzene polymer, for example a halogenated (e.g. brominated) styrene-divinyl benzene polymer such as Diaion SP207 (Mitsubishi); an anion exchanger (e.g. IRA-35 or IRA-68) an organic solvent-compatible cross-linked dextran such as Sephadex LH20 (Pharmacia UK Ltd), or on reverse phase supports such as hydrocarbon linked silica e.g. C₁₈- linked silica. An alternative chromatographic means for the purification/separation of the products of the fermentation process is countercurrent chromatography using a coil extracter such as a multi-layer coil extracter.

The products of the fermentation process may also be isolated and purified by the use of a liquid anion exchanger such as LA 2.

When IRA-68 or, particularly, IRA-35 is used as the solid adsorbant the cell extracts may be loaded directly without removal of solvent. The extract may either be loaded directly at about pH3 or at about pH8 following filtration of solid impurities.

Suitable solvents/eluants for the chromatographic purification/ separation of appropriate compounds of formula (IV) will, of course, depend on the nature of the column type and support. When using countercurrent chromatography we have found a solvent system comprising ethyl acetate, hexane, methanol and an aqueous acid (e.g. aqueous sulphuric acid) to be particularly suitable. When using an anion exchanger such as IRA-35 the resin may conveniently be washed with aqueous acetone followed by elution with sulphuric acid in aqueous acetone.

The presence of the products of the fermentation process during the extraction/isolation procedures may be monitored by conventional techniques such as h.p.l.c. or UV spectroscopy or by utilising the properties of the compounds.

Where a product of the fermentation process is obtained in the form solution in an organic solvent, for example after purification by chromatography, the solvent may be removed by conventional procedures, e.g. by evaporation, to yield the required compound. If desired, the compound may be further purified by the aforementioned chromatographic techniques.

Acylation to provide a compound of formula (IV) in which R¹ represents an acyloxy group as defined in formula (I) above may be effected by treating a corresponding compound of formula (IV) in which R¹ is a hydroxyl group or a protected derivative thereof with a suitable acylating agent under conventional esterification conditions followed by removal of any protecting groups present. Thus, for example, when R¹ in formula (IV) represents 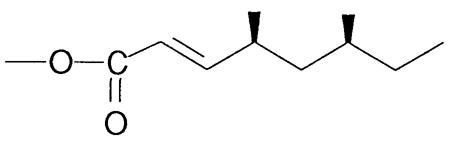
the R¹ group may be introduced by treating a compound of formula (IV) in which R¹ is a hydroxy group with an acid of formula (V) 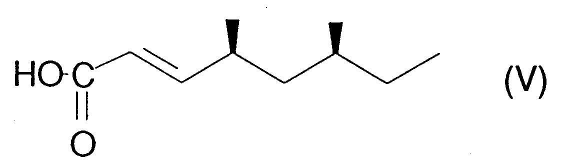
or an activated derivative thereof such as the corresponding acid chloride. The acylation reaction may conveniently be carried out in the presence of 4-dimethylaminopyridine with or without a suitable base such as a tertiary amine (e.g.triethylamine) or using an alkali metal carbonate or an alkaline earth metal carbonate (e.g. calcium carbonate) in a solvent such as a halogenated hydrocarbon (e.g. dichloromethane).

It is to be understood that the acylation or deacylation and esterification processes may be combined as sequential or simultaneous reaction steps as appropriate.

The compound of formula (V) may conveniently be prepared by hydrolysis of a compound of formula (IV) in which R¹ represents 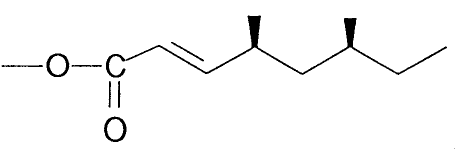
for example by base catalysed hydrolysis using a base such as aqueous sodium hydroxide in a solvent such as an alcohol (e.g. methanol).

Salts of compounds of formula (I) may be conveniently formed by treating a compound of formula (I) with an appropriate salt or base. Thus, for example, salts may conveniently be prepared by treating a compound of formula (I) with a salt or a base selected from sodium or potassium hydroxide, hydrogen carbonate, carbonate or acetate (e.g. potassium hydroxide, potassium hydrogen carbonate, sodium hydrogen carbonate or potassium acetate), ammonium acetate, calcium acetate and L-lysine as appropriate. The salt may, for example, be prepared by adding the appropriate salt or base (if necessary as an aqueous solution) to a solution or suspension of the compound of formula (I) in a suitable solvent such as water and/or a cosolvent such as an alcohol (e.g. methanol) or a nitrile (e.g. acetonitrile) at temperatures of for example 0⁰C to 80⁰C and conveniently at about room temperature.

Physiologically acceptable salts may also be prepared from other salts, including other physiologically acceptable salts of the compounds of formula (I) using conventional methods.

Compounds of formula (III) are novel intermediates and form a further aspect of the present invention.

The following examples are provided by way of illustrating the invention and are not intended to limit the invention in any way.

### Intermediate 1

### [1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1] octane-3,4,5-tricarboxyic acid, 6-(4,6-dimethyl- 2-octenoate)

(a) IMI 332962 was grown on agar plates of the following composition:

| | |
|---|---|
| Malt extract (Oxoid L39) | 30g |
| Mycological peptone (Oxoid L40) | 5g |
| Yeast extract (Oxoid L21) | 0.5g |
| Agar (Oxoid No 3) | 20g |
| Distilled water to 1 litre | |

| | | |
|---|---|---|
| Seed medium (A): | Peptone (Oxoid L34) | 10g |
| | Malt extract (Oxoid L39) | 21g |
| | Glycerol | 40g |
| | Junlon 110 (Honeywill & Stein Ltd., Wallington, Surrey) | 1g |
| | Distilled water to 1 litre | |

The pH of the medium was adjusted to 6.3-6.5 with aqueous sodium hydroxide before autoclaving
The flasks of inoculated seed medium were incubated at 25°C on a shaker platform, which rotated at 250rpm with a 50mm diameter orbital motion, for 5 days.
The contents of the flasks were pooled and homogenised. The homogenised seed culture was used at 3% (v/v) to inoculate 120, 50ml aliquots of fermentation medium (B) in 250ml Erlenmeyer flasks:

| | | |
|---|---|---|
| Fermentation medium (B): | Glycerol | 50g |
| | Soyabean oil | 30g |
| | Cottonseed flour (Sigma) | 10g |
| | Distilled water to 1 litre | |

The pH of the medium before autoclaving was in the range 6.1- 6.3. The flasks were incubated as above with shaking for 8 days.
The fermentation broth (approximately 6L) from flasks incubated for 8 days was filtered to remove the mycelium and the filtrate adjusted to pH 2.8 with sulphuric acid (20% v/v) and extracted with 3 x 2 volumes of ethyl acetate. The ethyl acetate extracts were bulked and back extracted with 2 x 400ml of aqueous sodium hydrogen carbonate solution (1% w/v). The aqueous back extracts were bulked, adjusted to pH 2.8 as above and re-extracted into 2 x 800ml of ethyl acetate. These extracts were combined and evaporated to dryness to yield a brown oil. This oil was further processed by countercurrent chromatography using an Ito Multi-layer Coil Extractor (P. C. Inc., Potomac, Maryland, USA). The coil used was the standard preparative coil consisting of approximately 70 metres of 2.6mm internal diameter PTFE tubing giving a total volume of about 380ml. The solvent system used was a mixture of ethyl acetate, hexane, methanol and N/100 sulphuric acid (6:5:5:6 by volume). The lower phase was kept stationary. The coil was filled with the lower phase using a Gilson Model 303 pump and a Model 804C Manometric Module (Gilson, Villiers Le Bel, France). The oil (497mg in 4ml of the upper phase +4ml of the lower phase) was then injected at the "tail" end of the column. The centrifuge was then operated at 800 rev./min. and the mobile (upper) phase pumped at 4ml/min. from the "tail" end of the column. 20ml fractions were collected and monitored by measuring inhibition of squalene synthase.
Consecutive fractions showing activity against squalene synthase were bulked. The earlier fractions were evaporated to dryness to yield the title compound (90mg) as a pale yellow oil.
(b) The mycelium separated from 6L broth, from flasks incubated for 8 days according to the procedure in part (a) above, was extracted with methanol (2 x 3L) and filtered. The filtrate was concentrated by evaporation to ca. 500ml, adjusted to pH 3.0 with formic acid and extracted with 3 x 500ml of ethyl acetate. The ethyl acetate extracts were bulked and back extracted with 2 x 200ml of sodium hydrogen carbonate solution (1% w/v). The aqueous back extracts were bulked, adjusted to pH 3.0 and re-extracted into 2 x 500ml of ethyl acetate. All the organic fractions were combined and reduced to dryness using a rotary evaporator to yield a brown oil. The oil (578mg) was further processed by high peformance liquid chromatography (HPLC) using a Gilson autopreparative system composed of 3 Gilson solvent delivery pumps (model 303), an 811 Dynamic mixer and an 802C manometric module. The chromatography was carried out on a Dynamax Microsorb C18 (5µm) semi-preparative column (250 x 10mm). The mobile phase was a gradient composed of acetonitrile and 0.1% v/v formic acid to pH 3.15 with ammonium acetate (1:3 → 4:1 → 1:3) pumped at 2.8-5.6ml/min with a run time of 65 minutes. This method was repeated 16 times. 13 x 4.95 minute fractions were collected and monitored by measuring inhibition of squalene synthase. Fraction number 5 from each run was bulked, acidified to pH 3.0 with formic acid and extracted with 2 x 100ml ethyl acetate. The organic phase was removed and evaporated to dryness to yield the title compound (172mg) as a pale yellow oil.
(c) (i) Eight 0.5ml aliquots from a 5 day old fermentation carried out as in part (a) above were used to inoculate eight 50ml aliquots of seed medium (A) contained in 250ml Erlenmeyer flasks. The flasks were incubated at 25°C on a shaker platform, which rotated at 250rpm with a 50mm diameter orbital motion, for 4 days. The contents of the flasks were pooled and homogenised.
The homogenised seed culture was used at 3% (v/v) to inoculate 120, 50ml aliquots of fermentation medium (B) in 250ml Erlenmeyer flasks. The flasks were incubated with shaking as above for 10 days.
(c) (ii) Homogenised seed culture prepared as in part(c)(i) above were used at 3% (v/v) to inoculate two fermentation vessels, each of 5 litres capacity, containing 3 litres of fermentation medium (B). The inoculated medium was maintained at 25°C and agitated with two six bladed turbine impellers (70mm diameter) rotating at 500 rpm. The culture was aerated by sparging with sterile air at 3 Lpm. Provision was made for control of excessive foaming of the culture by the addition of silicone antifoam (Dow Corning 1520). The contents of the two culture vessels were combined after 11 days growth and further processed by countercurrent chromatography according to the procedure in part (a) above to give the title compound (137mg); 500MHz proton nmr in deutero-methanol includes signals at about δ 0.84-0.90 (m,9H), 1.03 (d,7,3H), 1.09-1.19 (m,2H), 2.10 (s,3H), 2.24 (m,1H), 2.34 (m,1H), 2.68 (dd,13,6,1H), 4.04 (d,2,1H), 4.97 (s,1H), 5.02 (s,1H), 5.08 (d, 5,1H), 5.27 (s,1H), 5.80 (d,16,1H), 6.31 (d,2,1H), 6.85 (dd,16,8,1H), 7.14 (t,7,1H), 7.19 (d,7,2H), 7.26 (t,7,2H); composite pulse decoupled 125.75 MHz carbon-13 nmr in deutero-methanol includes peaks at about δ 172.5 (0), 172.1(0), 170.1(0), 168.5(0), 166.5 (0), 157.6 (1), 147.7 (0), 141.6 (0), 130.2 (1), 129.3 (1), 126.9 (1), 119.8 (1), 111.5 (2), 106.8 (0), 91.1 (0), 82.5 (1), 81.0 (1), 80.1 (1), 76.6 (1), 75.6 (0), 44.4 (2), 40.9 (2), 37.7 (1), 35.6 (1), 34.9 (2), 33.1 (1), 30.8 (2), 26.5 (2), 20.9 (3), 20.5 (3), 19.2 (3), 14.1 (3), 11.4 (3).
(d) (i) Frozen stocks of inoculum were prepared from a 5 day old fermentation carried out as in part (a) above. Samples of culture were centrifuged for 10 min and the mycelium resuspended to the original volume in 15% glycerol and 0.01% Tween 80. The mycelium was spun down and resuspended again before being distributed in 1.8ml amounts in plastic tubes and stored at -20°C. Eight 0.5ml aliquots of frozen inoculum were used to inoculate eight 50ml aliquots of seed medium (A) contained in 250ml Erlenmeyer flasks. The flasks were incubated at 25°C on a shaker platform, which rotated at 250rpm with a 50mm diameter orbital motion, for 4 days. The contents of the seed flasks were pooled and used at 3% (v/v) to inoculate 120 50ml aliquots of fermentation medium (B) in 250ml Erlenmeyer flasks. The flasks were incubated with shaking as above for 9 days.
(d) (ii) The contents of 4 final stage flasks grown as in part (d)(i) above were pooled after 7 days incubation and homogenised to provide the seed for 120 50ml aliquots of fermentation medium (B) which were incubated for 8 days as in parts (c)(i) and(d)(i) above. The fermentation broth (approximately 6L) from flasks incubated for 8 days was filtered to remove the mycelium. The filtrate was adjusted to pH 2.8 with sulphuric acid (20% v/v) and extracted into ethyl acetate, back extracted into sodium hydrogen carbonate and re- extracted into ethyl acetate at pH 2.8 as described in part (a) above. The ethyl acetate extract was concentrated under reduced pressure to a yellow oil which was dissolved in methanol (10ml). This solution was evaporated to 3ml and applied to a column (32x 2.5cm) of ODS-3 (Whatman Partisil Bioprep 40,75 Angstrom, slurry packed in acetonitrile-water, 20:80). The column was eluted with a stepwise gradient of a mixture of acetonitrile and water, increasing the proportion of acetonitrile as follows: 1:4, 3:7, 2:3, 1:1, 3:2. Fractions were monitored by HPLC and those containing the title compound were evaporated to remove acetonitrile. The resulting aqueous suspensions were pooled and freeze dried overnight to yield the title compound (59mg) as an off-white solid.
(e) The procedure in part (d)(i) was followed except that the pooled seed flasks were used at 3% (v/v) to inoculate 4 litres of seed medium (A) in a 7L fermenter. The culture was incubated with agitation as above at 500rpm for 2 days with the culture aerated at 4L/min. 1.2L of the culture was removed and used to inoculate a 70L fermenter filled with 40L seed medium (A). The culture was incubated as above at 500rpm for 2 days with the culture aerated at 40 L/min. 15L of the culture was removed and added to a 780L fermenter filled with 500L fermentation medium (C).

| | | |
|---|---|---|
| Fermentation medium (C) : | Fructose | 50g |
| | Soyabean oil | 30g |
| | Cottonseed flour (sigma) | 20g |
| | Natural pH | |

The culture was incubated with shaking as above at 200rpm for 450h with the culture aerated at 500L/min and fed at 120h with a 50% (w/v) solution of fructose at 5g/L/day increasing to 7.5g/L/day at 162h. Analysis of the broth at 450h indicated a yield of the title compound of 1056 mg/L.

The above procedure was repeated on a reduced scale but replacing fructose with other sources of carbon selected from glucose, galactose, sucrose, maltose, lactose, myo-inositol, D- mannitol and soyabean oil. Analysis of the broth from each experiment at 450h indicated a substantial titre of the title compound.

The title compound prepared according to the above procedures was consistent with a product having the following characterising features:
Approximate molecular weight 690; -FAB mass spectrometry [M-H]- 689.2789; +FAB mass spectrometry [M+Na}+ 713.2753; Molecular formula C₃₅H₄₆O₁₄.

500 MHz proton nmr spectrum in deutero-chloroform [ δ values with multiplicities, coupling constants (Hz) and integration values in parenthesis] : 0.79 to 0.85 (m,9H), 1.00 (d,7,3H), 1.04 to 1.15 (m,2H), 2.09 (s,3H), 2.40 (m,1H), 2.69 (dd,13,5,1H), 4.05 (s,1H), 4.94 (s,1H), 4.96 (s,1H), 5.06 (d,4,1H), 5.30 (s,1H), 5.78 (d,16,1H), 5.92 (s,1H), 6.88 (dd,16,8,1H), 7.11 (d,7,2H), 7.14 (t,7,1H), 7.24 (t,7,2H).

Composite pulse decoupled 125.75MHz carbon-13 nmr spectrum in deutero-chloroform [δ values with the number of attached protons in parenthesis]: 171.5 (0), 171.0(0), 169.1(0), 167.0(0), 166.7(0), 157.9(1), 145.4(0), 140.1(0), 128.9(1), 128.1(1), 125.8(1), 117.8(1), 111.4(2), 105.8 (0), 88.5 (0), 81.6 (1), 80.7 (1), 79.3 (1), 75.1 (1), 74.2 (0), 42.9 (2), 39.7 (2), 36.7 (1), 34.2 (1), 33.6 (2), 31.6 (1), 29.4 (2), 25.4 (2), 20.9(3), 19.8(3), 18.8(3), 13.5(3), 10.9(3).

### Intermediate 2

### [1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8- dioxabicyclo[3.2.1] octane-3,4,5-tricarboxyic acid, 6-(4,6-dimethyl- 2-octenoate),3,4,5-trimethyl ester

A solution of the freeze dried product of Intermediate 1 (940mg) in methanol (15ml) was treated with a solution of diazomethane in diethyl ether (0.4M; 16ml). The excess diazomethane was quenched with acetic acid (0.1ml) and the solution was concentrated under reduced pressure. The residue was chromatographed on silica gel (Merck 7734, 50g) eluting with dichloromethane increasing to 2% methanol/dichloromethane to give the title compound (906mg); δ(CDC1₃) includes 0.8-0.9 (m,CH₃), 1.04 (d,J7Hz,CH=CHCHCH₃), 2.09 (s,CH₃CO₂), 3.76, 3.81 and 3.93 (3s,CO₂CH₃), 4.05 (d,J2Hz,7-H), 4.98 and 5.00 (2s,C=CH₂), 5.10 (d,J6Hz,CHOAc), 5.26 (s,3-H), 5.75 (d,J16Hz,CH=CHCO₂), 5.81 (d,J2Hz,6-H), 6.84 (dd,J₁16Hz,J₂8.5Hz,CH=CHCO₂), 7.13-7.28 (m,aromatic).

### Intermediate 3

### [1S-[1α4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 7-acetate, 6-(4,6-dimethyl-2-octenoate), 3,4,5-trimethyl ester

A solution of Intermediate 2 (10.84g) and dimethylaminopyridine (228mg) in dichloromethane (163ml) was treated with triethylamine (10.8ml) and acetic anhydride (4.4ml) under nitrogen at 0°C. The reaction mixture was stirred for 3.5h and then treated with aqueous saturated sodium bicarbonate solution (100ml). The mixture was extracted with dichloromethane (2 x 250ml) and the combined organic phases were dried, concentrated and chromatographed on silica gel (Merck 9385) eluting with ethyl acetate: cyclohexane (1:4 increasing to 1:1) to give the title compound (10.53g); δ (CDC1₃) includes 1.03 (d,J6Hz,=CHCHCH₃), 2.1, 2.19 (2s,2AcO), 3.74, 3.79, 3.98 (3s,CO₂CH₃), 5.11 (d,J5Hz,CHOAc), 5.12 (s,3-H), 5.28 (d,J2Hz,7- H), 5.72 (d,J15Hz,=CHCO₂), 6.33 (d,J2Hz,6-H), 6.83 (dd,J 15 and 8Hz, CH=CHCO₂), 7.1-7.3 (m,C₆H₅);

| | | |
|---|---|---|
| Analysis found: | C,62.11; | H,6.99 |
| C₄₀H₅₄O₁₅requires: | C,62.00; | H,7.02%. |

### Intermediate 4

### [1S-1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 7-acetate,6-(4,6-dimethyl-2-octenoate), 4-[[(1,1-dimethylethyl)thio]2-oxo- acetate],3,4-trimethyl ester

A solution of Intermediate 3 (6.00g) in dry toluene (40ml) was treated with 4-dimethylaminopyridine (0.100g), dry triethylamine (3.8ml) and oxalyl chloride (2.0ml). After 4h stirring at room temperature, additional oxalyl chloride (1.0ml) was added. After a further 18h at room temperature, additional portions of dry triethylamine (2.0ml) and oxalyl chloride (1.0ml) were added. After a further 4 days stirring at room temperature, the mixture was concentrated and the resultant dark brown cake azeotroped with dry toluene. The brown cake was slurried into dry toluene (60ml) and treated with 4-dimethylaminopyridine (0.100g), dry triethylamine (0.38ml) and 2-methyl-2-propanethiol (1.04ml). The mixture was heated at 80°C for 3.5h, allowed to cool, then concentrated to a brown cake which was azeotroped with toluene. The residue was purified by flash column chromatographyon silica gel eating with ethyl acetate:cyclohexane ((1:4), (1:3), (7:13)), and appropriate fractions were combined and concentrated to give the title compound (2.97g); Rf 0.74 (silica gel, ethyl acetate-cyclohexane, (1:1)); δ (CDC1₃) includes 1.02(d,J7Hz, =CHCHCH₃), 1.47(s,C(CH₃)₃), 2.10 and 2.18(2s,2xO₂CCH₃), 2.70(dd,J13 and 5Hz,CHPh), 3.77, 3.85 and 3.92(3s,3xCO₂CH₃), 4.98 and 5.00(2s,C=CH₂), 5.10(d,J5Hz,CHOAc), 5.19(s,3-H), 5.28(d,J2.5Hz,7-H), 5.72(d,JI6Hz,O₂CCH=CH), 6.49(d,J2.5Hz,6-H), 6.85(dd,J16 and 8Hz,O₂CCH=CH), 7.1-7.3(m,C₆H₅).

### Intermediate 5

### [1S-[1α(4R*,5S*),3α,4β,5α,6α(2E,4R*,6R*),7β]] 1-(4-Acetyloxy-5-methyl-3-methylene-6-phenylhexyl)-4,6-dihydroxy-7[(2-methoxyethoxy)methoxy]-2,8-dioxabicyclo[3.2.1]octane-3,4,5- tricarboxylic acid, 6-(4,6-dimethyl-2-octenoate),3,4,5-trimethyl ester

A solution of Intermediate 2 (18.517g) in dichloroethane (280ml) was treated with N,N-diisopropylethylamine (17.8ml) followed by 2-methoxyethoxymethyl chloride (11.6ml) the latter in 2 portions over 15 minutes. The mixture was heated at 70°C for 43h and then allowed to cool. The solution was diluted with ethyl acetate (1000ml) and was then washed successively twice with aqueous hydrochloric acid (2M, 700ml), once with saturated aqueous sodium hydrogen carbonate solution (900ml), and once with water (900ml), then dried (MgSO₄), filtered and concentrated in vacuo to a yellow gum. The gum was purified by flash column chromatography on silica gel eluting with ethyl acetate:cyclohexane ((3:7), (1:1)) and appropriate fractions were combined and concentrated to give the title compound (17.758g) as a yellow oil. Rf 0.25 (silica gel, ethyl acetate-cyclohexane (1:1)); δ (CDCl₃) includes 1.02(d,J7Hz,=CHCH(CH₃), 2.10(s,O₂CCH₃); 2.72(ddJ13 and 5Hz,CHPh), 3.34(s,OCH₂CH₂OCH₃), 3.70, 3.76 and 3.95(3s,3xCO₂CH₃), 4.98 and 5.01(2s,C=CH₂), 5.22(s,3-H), 5.71(d,J16Hz,O₂CCH=CH), 6.40(bs,6-H), 6.83(dd,J16 and 8Hz,O₂CCH=CH), 7.1-7.3(m,C₆H₅).

### Intermediate 6

### [1S-[1α(4R*,5S*),3α,4β,5α,6a(2E,4R*,6R*),7β]] 1-(4-Acetyloxy-5-methyl-3-methylene-6-phenylhexyl)-6-hydroxy,7-[(2-methoxyethoxy)methoxy]-2,8-dioxabicyclo[3.2.1]octane-3,4,5- tricarboxylic acid,6-(4,6-dimethyl-2-octenoate),3,4,5-trimethyl ester

A solution of Intermediate 5 (5.622g) in dry toluene (30ml) was treated with 4-dimethylaminopyridine (0.83g), triethylamine (4.3ml) and methyl oxalyl chloride (2.5ml) and heated at 55°C for 187.5h. It was then allowed to cool and concentrated in vacuo to a brown residue. This was slurried into dry toluene (30ml), heated to 95°C, then treated with 2,2'-azobis(2-methylpropionitrile) (2 level spatula-fulls) and tris(trimethylsilyl)silane (8.0ml). After 5h, a further portion of 2,2'-azobis(2-methylpropionitrile) (1 level spatula-full) was added. After a further 19h at 95°C further portions of 2,2'-azobis(2-methylpropionitrile) (2 level spatula- fulls) and tris(trimethylsilyl)silane (4.0ml) were added. After an additional 7h at 95°C, more 2,2'-azobis(2-methylpropionitrile) (1 level spatula-full) was added, and after another 17h, the mixture was allowed to cool. The mixture was purified directly by flash column chromatography on silica gel eluting with ethyl acetate:cyclohexane ((1:4), (3:7), (1:1)) and appropriate fractions were combined and concentrated to a brown gum. This was further purified by flash column chromatography on silica gel eluting with ethyl acetate:cyclohexane ((1:3), (3:7), (1:1)) and appropriate fractions were combined and concentrated in vacuo to give the title compound (0.206g) as a yellow film; Rf 0.44 (silica gel, ethyl acetate-cyclohexane, (1:1)); δ (CDCl₃) includes 1.01(d,J7Hz,=CHCH(CH₃), 2.10(s,O₂CCH₃), 2.72(dd,J13 and 5Hz,CHPh), 3.09(d,J12Hz,4-H), 3.34(s,OCH₂CH₂OCH₃), 3.70 and 3.76(2s,3xCO₂CH₃), 4.11(d,J2Hz,7-H), 5.08-5.15(m,3-H and CHOAc), 5.70(d,J16Hz,O₂CCH=CH), 6.23(d,J2Hz,6-H), 6.82(dd,J16 and 8Hz,O₂CCH=CH), 7.1-7.3(m,C₆H₅); RT 27.05 minutes on reverse-phase HPLC (25x0.4cm, Spherisorb, ODS-2) eluting with acetonitrile-water (33.25% to 90.25% over 23 minutes) acidified with 0.15ml 1⁻¹ conc H₂SO₄.

### Intermediate 7

### [1S-[1α(4R*,5S*),3α,4β,5α,6α(2E,4R*,6R*),7β]] 1-(4-Acetyloxy-5-methyl-3-methyiene-6-phenylhexyl)-6-hydroxy-7-[(2-methoxyethoxy)methoxy]-2,8-dioxabicyclo[3.2.1]octane-3,4,5- tricarboxylic acid,6-(4,6-dimethyl-2-octenoate)

A solution of Intermediate 6 (0.062g) in dimethylsulphoxide (1.6ml) and water(7 drops) was treated with lithium iodide (0.103g) and heated with stirring at 145°C. After 122 hours at 145°C, and 2 days at room temperature, the mixture was diluted with ethyl acetate (20ml) and saturated aqueous sodium chloride solution (20ml). The organic phase was separated and the aqueous phase extracted three times with ethyl acetate (20ml) and solid sodium chloride added. The combined organic phases were dried (MgSO₄), filtered and concentrated to a dark brown film. This was purified by reverse- phase HPLC (1 inch, Spherisorb, ODS-2) eluting with acetonitrile- water (47.5% to 90.25% over 20 minutes) with 0.15ml 1⁻¹ concentrated sulphuric acid. Appropriate fractions were combined and concentrated in vacuo to remove acetonitrile. The aqueous solution was extracted three times with ethyl acetate. The combined organic phases were dried (MgSO₄), filtered and concentrated to give the title compound (0.010g) as a brown film; δ (CD3OD) includes 1.03 (d,J6Hz,=CHCH(CH3)), 2.10 (s,O2CCH3), 2.68 (dd,J13 and 6Hz,CHPh), 3.01 (d,J11Hz,4-H), 3.45-3.53 and 3.70-3.80 (2m,OCH2CH2O), 4.11 (d,J2Hz,7-H), 4.96 and 5.00 (2bs,C=CH2), 5.06 (d,J5Hz,CHOAc), 5.82 (d,J15Hz,O2CCH=CH), 6.15 (bs,6-H), 6.87 (dd,J15 and 8Hz,O2CCH=CH), 7.1-7.3 (m,C6H5); RT 18.32 minutes on reverse-phase HPLC (25x0.4cm,Spherisorb,ODS-2) eluting with acetonitrile-water (33.25% to 90.25% over 23 minutes) acidified with 0.15ml 1⁻¹ concentrated sulphuric acid.

### Intermediate 8

### [1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl- 2-octenoate), 3,4,5-tris(1,1-dimethylethyl) ester

Freeze-dried Compound A of Intermediate 1 (7.5g) in dry dichloromethane (32ml) was heated at reflux, under nitrogen and treated dropwise over 20 min with N,N-dimethylformamide di- tert.butyl acetal (31.3ml). The mixture was heated under reflux for 1h when a further addition of N,N-dimethylformamide di-tert.butyl acetal (7.21ml) was made over 3 min. The mixture was heated under reflux for a further 4h and was then allowed to cool to room temperature, diluted with diethyl ether (200ml) and washed with brine (3x100ml). The organic phase was dried (MgSO4) and evaporated to give a red-brown foam (10.75g). This was subjected to flash chromatography on silica gel (Merck 9385, 1100ml) eluting with ethyl acetate:cyclohexane (1:6). Fractions which contained the major component were combined and evaporated to give the title compound (6.55g) as a cream-yellow foam; v max (CHBr₃) ca 3400-3600 (OH), 1755 (ester C=0), 1730 (ester C=0) and 1250cm-1 (ester C=0); (CDCl₃) includes 1.43(s,Me₃C-), 1.48(s,Me₃C-), 1.60(s,Me₃C-), 2.08(s,CH₃CO₂- ), 2.93(d,J=3Hz,7-OH), 4.00(broad s,7-H), 4.08(s,4-OH), 4.95(bs,C=CH₂), 5.05(s,3-H), 5.11(d,J=5Hz,CH₃CO₂CH), 5.77(d,J=15Hz,CH=CH.CHMe), 6.01(d,J=2Hz,6H), 6.91(dd,J=15Hz,7Hz,CH=CH.CHMe) and 7.10-7.30(m,aromatic protons).

### Intermediate 9

### [1S-[1α(4R*,5S*),3α,4β,5α,6α(2E,4R*,6R*), 7β(2R*,2S*)]] 1-(4-Acetyloxy-5-methyl-3-methylene-6-phenylhexyl)-4,6-dihydroxy-7-(2-tetrahydropyranyloxy)-2,8-dioxabicyclo[3.2.1]octane-3,4,5- tricarboxylic acid,6-(4,6-dimethyl-2-octenoate),3,4,5-tris(1,1- dimethylethyl)ester

A solution of Intermediate 8 (6.60g) in dichloromethane (150ml) was treated with 3,4- dihydro-2H-pyran (15ml) and pyridinium p-toluenesulphonate (1g). After 20h stirring at room temperature, the mixture was diluted with dichloromethane (220ml) and washed with a saturated solution of sodium hydrogen carbonate (100ml) and a saturated solution of ammonium chloride (100ml). The organic phase was dried and concentrated to give a brown oil. This was purified by flash column chromatography on silica gel eluting with ethyl acetate:cyclohexene (2:8) to give the title compound (6.10g); δ (CDCl₃) includes 1.02 (d,J =7Hz, = CHCHCH₃), 1.40, 1.42, 1.45, 1.65 and 1.70 (5s, COOC(CH₃)₃), 2.1 (s, OCOCH₃), 2.75 (dd, J =5 and 13Hz, CHCH₂-Ph), 4.05 and 4.08 (s, OH, 4.08 and 4.21 (d, J = 2Hz, 7-H), 5.07 and 5.10 (s, 3-H), 5.15 (d, J =5Hz, CH₃COO-CH, 5.75 (d, J= 15.5Hz, OOC-CH=), 6.45 and 6.60 (d, J =2Hz, 6-H), 6.90 (dd, J = 8 and 16Hz, O₂CCH=CH), 7.1-7.3 (m, C₆H₅);

| | | |
|---|---|---|
| Analysis Found: | C,66.27; | H,8.56. |
| C₅₂H₇₈O₁₅ requires: | C,66.22; | H,8.34%. |

### Intermediate 10

### [1S-[1α(4R*,5R*),3α,4β,5α,6α(2E,4R*,6R*),7β(2R*,2S*)]] 1-(4-Acetyloxy-5-methyl-3-methylene-6-phenylhexyl)-4,6-dihydroxy-7-(2-tetrahydropyranyloxy)-2,8-dioxabicyclo[3.2.1]octane-3,4,5- tricarboxylic acid,6-(4,6-dimethyl-2-octenoate),4-[(methoxy)-2-oxo-acetate],3,4,5-tris(1,1-dimethylethyl)ester

A solution of Intermediate 9 (5.20g) in dry tetrahydrofuran (80ml) was treated with sodium hydride (0.38g) at room temperature. After 10 min methyl oxalyl chloride (1.3ml) was added. After 10 min a saturated solution of ammonium chloride (100ml) and diethyl ether (200ml) were added. The organic phase was dried and evaporated to give the title compound (5.2g); δ (CDCl₃) includes 1.02 (d,J = 7Hz, = CHCHCH₃), 1.42, 1.45, 1.55 and 1.60 (4s, COOC(CH₃)₃), 2.1 (s, OCOCH₃), 3.8 (s, CH₃OCOCOO), 4.70 and 4.82 (bt, O-CH-O), 4.95 (bs, CH₂=), 5.21 (d, J = 5Hz, CH₃COO-CH), 5.80 (d, J = 15.5Hz, O₂C-CH=), 6.40 and 6.52 (d,J = 2Hz, 6-H), 6.90 (dd, J = 8 and 15.5Hz, O₂CCH=CH), 7.10-7.30 (m, C₆H₅).

### Intermediate 11

### [1S-[1α(4R*,5S*),3α,4β,5α,6α(2E,4R*,6R*), 7β(2R*,2S*)]] 1-(4-Acetyloxy-5-methyl-3-methylene-6-phenylhexyl)-6-hydroxy-7(2-tetrahydropyranyloxy)-2,8-dioxabicyclo[3.2.1]octane-3,4,5-tricarboxylic acid,6-(4,6-dimethyl-2-octenoate),3,4,5-tris(1,1-dimethylethyl)ester

A solution of Intermediate 10 (5.1g) in dry xylene (80ml) at 90°C was treated with tributyltin hydride (2.2ml) and 2,2'-azo-bis(2-methylpropionitrile) (50mg). Further portions of 2,2'-azo-bis (2-methylproprionitrile) (50mg) and tributyltin hydride (0.7ml) were added after 3h with continued heating at 90°C. After a total of 16h, heating was stopped, the reaction mixture was allowed to cool, then purified by flash column chromatography on silica gel eluting with ethy acetate:cyclohexane ((5:95), (1:9), (1:4)) to give the title compound (1.56g); δ (CDCl₃), 1.02 (d,J = 6.5Hz, = CHCHCH₃), 2.1 (s, CH₃COO), 2.95 (d,J = 12Hz, 4-H), 4.02 and 4.20 (d, J = 2Hz, 7-H), 4.92 and 4.95 (bs, = CH₂), 4.94 and 4.98 (d, J = 12 Hz, 3-H), 5.10 and 5.15 (d, J = 5Hz, CH₃COOCHCH), 5.75 and 5.80 (d,J = 15Hz, O₂C-CH=CH), 6.23 and 6.38 (d, J = 2Hz, 6-H), 6.90 (dd, J = 7.5 and 15Hz), 7.1-7.3 (m, C₆H₅).

| | | |
|---|---|---|
| Analysis found: | C,67.38; | H,8.72. |
| C₅₂H₇₈O₁₄requires: | C,67.60; | H,8.51%. |

### Intermediate 12

### [1S-[1α(4R*,5S*),3α,4β,5α,6α(2E,4R*,6R*), 7β]] 1-(4-Acetyloxy-5-methyl-3-methylene-6-phenylhexyl)-6-hydroxy-2,8-dioxabicyclo[3.2.1]octane-3,4,5-tricarboxylic acid,6-(4,6-dimethyl-2-octenoate),3,4,5-tris(1,1-dimethylethyl)ester

A solution of Intermediate 11 (1.34g) in methanol (100ml) at 60°C was treated with pyridinium p-toluenesulphonate (0.1g). After 16h stirring at 60°C the solvent was evaporated and ethyl acetate (150ml) was added. The organic phase was washed with a saturated solution of sodium hydrogen carbonate (30ml) and a saturated solution of ammonium chloride (30ml). The organic phase was dried and evaporated to give the title compound (1.15g); δ (CDCl₃) includes 1.02 (d,J = 7Hz, = CHCHCH₃), 1.44, 1.45 and 1.47 (s, COOC(CH₃)₃), 2.10 (s, CH₃COOCH), 2.70 (dd, J =5 and 13Hz, CHCHC₆H₅), 2.95 (d,J = 11Hz, 4-H), 3.07 (bd, 7-OH), 3.98 (bs, 7-H), 4.90 (d, J = 11Hz, 3-H), 4.93 and 4.95 (bs, C=CH₂), 5.10 (d,J = 5Hz, CH₃COOCH), 5.72 (d, J = 2Hz, 6-H), 5.75 (d, J = 15Hz, O₂CCH = CH), 6.9 (dd, J = 7 and 15Hz, O₂CCH = CH-CH), 7.10-7.30 (m, C₆H₅).

### Example 1

### [1S-[1α(4R*,5S*),3α,4β,5α,6α(2E,4R*,6R*),7β]] 1-(4-Acetyloxy-5-methyl-3-methylene-6-phenylhexyl)-6,7-dihydroxy-2,8-dioxabicyclo[3.2.1]octane-3,4,5-tricarboxylic acid 7-acetate,6-(4,6-dimethyl-2-octenoate),3,4,5-trimethyl ester

A solution of Intermediate 4(1.56g) in dry toluene (40ml) at 90°C was treated with 2,2'-azobis(2-methylpropionitrile) (1 spatula-full) and tris(trimethylsilyl)silane (2.5ml). Further portions of 2,2'-azobis(2methylpropionitrile) (spatula-full) were added after 18h and 24h with continued heating at 90°C. After a total of 48h, heating was stopped, the reaction mixture was allowed to cool, then purified by flash column chromatography on silica gel eluting with ethyl acetate:cyclohexane((1:9), (3:7), (1:1)), and appropriate fractions were combined and concentrated to give the title compound (0.266g) as a clear colourless gum; Rf 0.35 (silica gel,ethyl acetate-cyclohexane, (1:1)); δ (CDCl₃) includes 0.8-0.9(m,CH₃), 1.01(d,J7Hz,=CHCH(CH₃), 2.10 and 2.20(2s,2xO₂CCH₃), 2.72(dd,J13 and 5Hz,CHPh), 3.12(d,J12Hz,4-H), 3.75, 3.78 and 3.79(3s,3xCO₂CH₃), 4.94 and 4.99(2bs,C=CH₂), 5.03(d,J12Hz,3-H), 5.10(d,J5Hz,CHOAc), 5.26(d,J2.5Hz,7-H), 5.71(d,J16HzO₂CCH=CH), 6.16(d,J2.5Hz,6-H), 6.82(dd,J16 and 8Hz,O₂CCH=CH), 7.1-7.3(m,C₆H₅).

### Example 2

### [1S-[1α(4R*,5s*),3α,4,5α,6α(2E,4R*,6R*),7β]] 1-(4-Acetyloxy-5-methyl-3-methylene-6-phenylhexyl)-6,7-dihydroxy-2,8-dioxabicyclo[3.2.1] octane-3,4,5-tricarboxyic acid,7-acetate, 6-(4,6-dimethyl-2-octenoate)

A solution of Example 1 (0.266g) in dimethylsulphoxide (7ml) and water (20 drops) was treated with lithium iodide (0.381g) and heated with stirring at 145°C. After 91h, the mixture was allowed to cool to room temperature, and was diluted with ethyl acetate (150ml). The organic solution was washed successively with saturated aqueous sodium thiosulphate solution (100ml), saturated aqueous sodium thiosulphate/sodium chloride solution (100ml) and saturated sodium chloride solution (100ml), then dried and concentrated to a dark brown film. This was purified by reverse- phase HPLC (1 inch Spherisorb, ODS-2) eluting with acetonitrile - water (47.5% to 90.25% over 20 minutes) acidified with 0.15ml 1-1 concentrated sulphuric acid. Appropriate fractions were combined and concentrated in vacuo to remove acetonitrile. The aqueous solution was extracted three times with ethyl acetate. The combined organic phases were washed with saturated aqueous sodium chloride solution, dried (MgSO₄), filtered and concentrated to give the title compound (0.081g) as a colourless film; δ (CD₃OD) include 1.03(d,J7Hz,=CHCH(CH₃), 2.09 and 2.18(2s,2xO2CCH₃), 2.67(dd,J13 and 5Hz,CHPh), 3.00(d,J12Hz,4-H, 4.91-5.00(m,3-H and C=CH₂), 5.05(d,J5Hz,CHOAc), 5.79(d,J16HzO₂CCH=CH), 6.19(d,J2Hz,6-H), 6.85(dd,J16 and 8Hz,O₂CCH=CH, 7.1-7.3(m,C₆H₅); RT 19.72 minutes on reverse-phase HPLC (15x0.4cm, Spherisorb, ODS-2) eluting with acetonitrile-water (14.25% to 90.25% over 25 minutes) acidified with 0.15ml 1⁻¹conc H₂SO₄;

| | | |
|---|---|---|
| Analysis found: | C,61.65; | H,7.14 |
| C₃₇H₄₈O₁₄requires: | C,62.00; | H,6.75%. |

### Example 3

### [1S-[1α(4R*,5S*),)3α,4β,5α,6α(2E,4R*,6R*),7β]] 1-(4-Acetyloxy-5-methyl-3-methylene-6-phenylhexyl)-6,7-dihydroxy-2,8-dioxabicyclo[3.2.1]octane-3,4,5-tricarboxylic acid, 7-acetate,6-(4,6-dimethyl-2-octenoate),3,4,5-tripotassium salt

A solution of Example 2 (0.068g) in 1,4 dioxan (11ml) was treated with aqueous potassium hydrogen carbonate solution (0.071M, 4.0ml). After 0.75h at room temperature, the solution was freeze-dried to give the title compound (0.080g) as a brown solid; δ (CD₃OD) includes 1.01(d,J7Hz,=CHCH(CH₃)) 2.10 and 2.13(2s,2xO₂CCH₃), 2.65-2.82(m,CHPh and 4-H), 4.95 and 5.01(2s,C=CH₂), 5.83(d,J16Hz,O₂CCH=CH), 6.55(d,J2Hz,6-H), 6.85(dd,J16 and 7Hz,O₂CCH=CH), 7.1-7.3(m,C₆H₅); RT 19.88 minutes on reverse-phase HPLC (15x0.4cm, Spherisorb ODS-2) eluting with acetonitrile-water (14.25% to 90.25% over 25 minutes) acidified with 0.15ml 1⁻¹ conc H₂SO₄.

### Example 4

**[1S-[1α(4R*,5S*),3α,4β,5α,6α(2E,4R*,6R*),7β]] 1-(4-Acetyloxy-5-methyl-3-methylene-6-phenylhexyl),6,7-dihydroxy-2,8-dioxabicyclo[3.2.1]octane-3,4,5-tricarboxylic acid,6-(4,6-dimethyl-2-octenoate),3,4,5-trimethyl ester**

A solution of Intermediate 6 (0.193g) in formic acid (9ml) was diluted with water (3ml) and heated at 65°C for 5h. The mixture was then allowed to cool, diluted with ethyl acetate (100ml) and poured into a conical flask where it was slowly neutralised by addition of saturated aqueous sodium hydrogen carbonate solution and solid sodium hydrogen carbonate. The two phases were then separated, the aqueous phase extracted with ethyl acetate (100ml), and the combined organic phases washed once with saturated aqueous sodium hydrogen carbonate solution (150ml), then dried (MgSO₄), filtered and concentrated to a clear colourless film. This was purified by reverse-phase HPLC (1 inch, Spherisorb, ODS-2) eluting with acetonitrile-water (80% to 95% over 10 minutes). Appropriate fractions were combined and concentrated in vacuo to remove acetonitrile. The aqueous solution was extracted three times with ethyl acetate and the combined organic phases dried (MgSO₄), filtered and concentrated to give the title compound (0.047g) as an off-white film; δ (CDCl₃) includes 1.02(d,J7Hz,=CHCH(CH₃)), 2.09(s,O₂CCH₃), 2.70(dd,J13 and 5Hz,CHPh), 3.11(d,J12Hz,4-H), 3.77 and 3.81(2s,3xCO₂CH₃), 4.01-4.04(m,7-H), 4.93 and 4.98(2s C=CH₂), 5.08-5.15(m,3-H and CHOAc), 5.66(d,J2Hz,6-H), 5.73(d,J16Hz,O₂CCH=CH), 6.82(dd,J16 and 8Hz,O₂CCH=CH), 7.1-7.3(m,C₆H₅); RT 23.96 minutes on reverse-phase HPLC (25x0.4cm, Spherisorb, ODS-2) eluting with acetonitrile-water (33.25% to 90.25% over 23 minutes) acidified with 0.15ml 1⁻¹ conc H₂SO₄.

### Example 5

### [1S-[1α(4R*5S*),3α,4β,5α,6α(2E,4R*,6R*),7β]] 1-(4-Acetyloxy-5-methyl-3-methylene-6-phenylhexyl)-6,7-dihydroxy-2,8-dioxabicyclo[3.2.1]octane-3,4,5-tricarboxylic acid,6(4,6-dimethyl- 2-octenoate)

A solution of Intermediate 7 (0.010g) in formic acid (0.9ml) was diluted with water (0.3ml) and heated at 65°C for 7h. The mixture was then allowed to cool and concentrated in vacuo to a brown film which was then azeotroped three times with toluene (3ml). It was then purified by reverse-phase HPLC (1 inch, Spherisorb, ODS- 2) eluting with acetonitrile-water (47.5% to 90.25% over 20 minutes) acidified with 0.15ml 1-1 conc H₂SO₄. Appropriate fractions were combined and concentrated in vacuo to remove acetonitrile. The aqueous solution was extracted twice with ethyl acetate (15ml). The combined organic phases were washed with saturated aqueous sodium chloride solution (30ml), dried (MgSO₄), filtered and concentrated to give the title compound (0.006g) as a brown film; δ (CD₃OD) includes 1.03 (d,J6Hz,=CHCH(CH₃)), 2.10 (s,O₂CCH₃), 2.66 (dd,J13 and 6Hz,CHPh), 3.02 (d,J11Hz,4-H), 4.02 (d,J2Hz,7-H), 4.95 and 5.00 (2bs,C=CH₂), 5.03-5.10 (m,3-H and CHOAc), 5.81 (d,J15Hz,O₂CCH=CH), 6.04 (d,J2Hz,6-H), 6.85 (dd,J15 and 8Hz,O₂CCH=CH), 7.1-7.3 (m,C₆H₅); RT 16.29 minutes on reverse-phase HPLC (25x0.4cm,Spherisorb,ODS2) eluting with acetonitrile-water (33.25% to 90.25% over 23 minutes) acidified with 0.15ml 1⁻¹ concentrated sulphuric acid.

### Example 6

### [1S-[1α(4R*,5S*),3α,4β,5α,6α(2E,4R*,6R*),7β]] 1-(4-Acetyloxy-5-methyl-3-methylene-6-phenylhexyl)-6,7-dihydroxy-2,8-dioxabicyclo[3.2.1]octane-3,4,5-tricarboxylic acid,6-(4,6-dimethyl-2-octenoate),3,4,5-tripotassium salt

A solution of Example 5 (0.010g) in 1,4-dioxan (2ml) was treated with aqueous potassium hydrogen carbonate solution (0.090M; 0.50ml). After 1h at room temperature, the solution was freeze-dried to give the title compound (0.011g) as a beige solid; δ CD₃OD) includes 1.03 (d,J6Hz,=CHCH(CH₃)), 2.10 (s,O₂CCH₃), 2.65-2.80 (m,4-H and CHPh), 4.06 (d,J2Hz,7-H), 5.09 (d,J5Hz,CHOAc), 5.84 (d,J16HzO₂CCH=CH), 6.38 (d,J2Hz,6-H), 6.87 (dd,J16 and 8Hz,O₂CCH=CH), 7.1-7.3 (m,C₅H₅); RT 16.19 minutes on reverse-phase HPLC (25x0.4cm,Spherisorb,ODS-2), eluting with acetonitrile-water (33.25% to 90.25% over 23 minutes) acidified with 0.15ml 1⁻¹ concentrated sulphuric acid.

### Example 7

### [1S-[1α(4R*,5S*)3α,4β,5α,6α(2E,4R*,6R*), 7β]] 1-(4-Acetyloxy-5-methyl-3-methylene-6-phenylhexyl)-6,7-dihydroxy-2,8-dioxabicyclo[3.2.1]octane-3,4,5-tricarboxylic acid,6-(4,6- dimethyl-2-octenoate) (Compound A) and [1S- [1α(4R*,5S*),3α,4β,5α,6α(2E,4R*,6R*),7β]] 1-(4-Acetyloxy-5- methyl-3-methylene-6-phenylhexyl)-6,7-dihydroxy-2,8-dioxabicyclo[3.2.1]octane-3,4,5-tricarboxylic acid,6-(4,6-dimethyl-2-octenoate),7-methanoate(Compound B)

A solution of Intermediate 12 (1.1g) in formic acid (10ml) was stirred at room temperature. After 16h the formic acid was evaporated and the residue was purified by reverse-phase HPLC (50mmx250mm Spherisorb ODS-2 column) eluting with 68% acetonitrile-water, acidified with 0.15ml 1⁻¹ concentrated sulphuric acid, flow rate 40 ml min⁻¹. Appropriate fractions were combined and concentrated in vacuo to remove acetonitrile. The aqueous solution was saturated with sodium chloride, acidified with hydrochloric acid (2M; 1ml) and extracted four times with ethyl acetate. The combined organic phases were washed with brine, dried (MgSO₄), filtered and concentrated to give title compound A (0.385g) having identical spectroscopic and analytical data to the title product of Example 5, and title compound B (0.121g); δ (CD₃OD) includes 2.1 (s, CH₃COO), 2.66 (dd,J=6 and 14Hz,CHC₆H₅), 3.02 (d,J=11Hz,4-H), 4.93 (d,J=11Hz,3-H), 5.05 (d,J=5Hz,CH₃COOCH), 5.27 (d,J=2Hz,7-H), 5.8 (d,J=15Hz,OCO-CH=CH), 6.2 (d,J=2Hz,6-H), 6.85 (dd,J=9 and 15Hz,CH=CH-CH), 7.1-7.3 (m,C₆H₅), 8.25 (s,OCOH),

| | | |
|---|---|---|
| Analysis Found: | C, 59.58; | H, 6.72; |
| C₃₆H₄₆O₁₄. 1.3 H₂O requires: | C, 59.54; | H, 6.75%. |

### Example 8

### [1S-[1α (4R*,5S*),3α,4β,5α,6α,7β]] 1-(4-Acetyloxy-5-methyl-3-methylene-6-phenylhexyl)-6,7-dihydroxy-2,8-dioxabicyclo[3.2.1]octane-3,4,5-tricarhoxylic acid

A solution of Example 5 (70mg) in dry dimethylformamide (2ml) was treated with triethylamine (0.063ml) and N-methylhyoxylamine hydrochloride (0.026g) at room temperature. After stirring for 16h the solvent was evaporated under reduced pressure. The residue was purified by reverse-phase HPLC (25mmx250mm Spherisorb, ODS-2 column) eluting with 43% acetonitrile-water acidified with 0.15ml 1⁻¹ concentrated sulphuric acid, flow rate 15ml min⁻¹. Appropriate fractions were combined and concentrated in vacuo to remove acetonitrile. The aqueous solution was saturated with sodium chloride, acidifled with hydrochloric acid (2M; 1ml), then extracted three times with ethyl acetate. The combined organic phases were washed with brine, dried (MgSO₄), filtered and concentrated to give the title compound (3Omg); δ (CD₃OD) includes 0.85 (d,J = 7Hz, CH₃CH), 2.10 (s,CH₃COO),2.7(dd,J = 6 and 14Hz, CHCHC₆H₅), 2.95 (d, J = 12Hz, 4-H), 4.0 (d, J = 1Hz, 7-H), 7.1-7.3 (m, C₆H₅).

| | | |
|---|---|---|
| Analysis found: | C,52.60; | H,6.26. |
| C₂₅H₃₀O₁₂.2.5 H₂O (567.6) requires: | C,52.91; | H,6.22%. |

### Example 9

### [1S-[1α(4R*,5S*),3α,4β,5α,6α(2E,4R*,6R*), 7β]] 1-(4-Hydroxy-5-methyl-3-methylene-6-phenylhexyl)-6,7-dihydroxy-2,8-dioxabicyclo[3.2.1]octane-3,4,5-tricarboxylic acid,6-(4,6-dimethyl-2-octenoate)

A solution of Example 5 (25mg) in acetone (0.75ml)-water (0.25ml) at room temperature was treated with concentrated sulphuric acid (0.05ml). After stirring 14 days the organic solvent was evaporated and the residue was purified by reverse-phase HPLC (25mmx250mm Spherisorb ODS-2column) eluting with 60% acetonitrile-water acidified with 0.15ml 1⁻¹ concentrated sulphuric acid, flow rate 15ml min⁻¹. Appropriate fractions were combined and concentrated in vacuo to remove acetonitrile. The aqueous solution was saturated with sodium chloride, acidified with hydrochloric acid (2M; 1ml), then extracted three times with ethyl acetate. The combined organic phases were washed with brine, dried (MgSO₄), filtered and concentrated to give the title compound (17mg); δ (CD₃OD) 1.0 (d, J = 7Hz, =CHCHCH₃), 2.75 (dd,J = 6 and 12.5Hz, CHC₆H₅), 3.05 (d,J = 11Hz, 4-H), 3.90 (d, J = 5Hz, =C-CH-OH), 4.03 (d,J = 1.5Hz, 6-H), 5.80(d,J = 15.5 Hz, O₂C-CH =), 6.85 (dd, J = 8.5 and 15.5Hz, O₂CCH=CH), 7.1-7.3 (m, C₆H₅).

| | | |
|---|---|---|
| Analysis Found: | C,60.11; | H,7.47 |
| C₃₃H₄₄O₁₂.1.5H₂O requires: | C,60.08; | H,7.18%. |

### Example 10

### Characteristics of IMI 332962

After 2-3 weeks growth at 25°C on oatmeal agar the colonies were smoke grey to mouse grey in colour (Rayner's Mycological Colour Chart, 1970; published by the Commonwealth Agricultural Bureaux) on both the surface and reverse of the colony.

Morphological observations of the strain grown at 25°C on oatmeal agar were made under an optical microscope. The fungus had no sexual reproductive stage but formed pycnidia, thereby placing it in the class Coelomycetes. The fungus produced rostrate pycnidia with loose hyphae and both aseptate and one-septate conidia. The conidia were approximately 5-9 x 1.5-3µM in dimensions (usually 7-9 x 1.502.5µM). Numerous multiseptate/multicellular, globose structures resembling chlamydospores or pycnidial initials were also observed. Distinct dictyochlamydospores were absent.

The isolate has been identified as a species of the genus Phoma, and the identity confirmed by the CAB International Mycological Institute.

## Claims

1. Compounds having the formula (I) 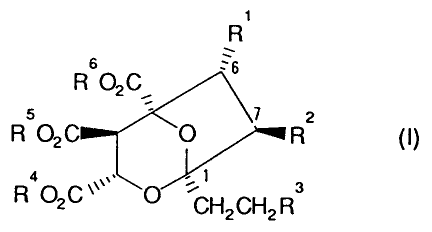 [wherein R¹ represents a hydroxyl group or a group selected from -OCOCH 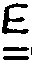 CHCH(CH₃)(CH₂)₃CH₃,-OCOCH 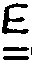 CHC(CH₃) 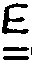 CHCH(CH₃)CH₂CH₃or -OCO-X-CH₂CH(CH₃)CH₂CH₃ [where X is -CH 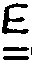 CHCH(CH₃)-, -CH₂CH(OH)CH(CH₃)-, - CH 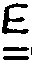 CHC(OH)(CH₃)-, -CH₂CH (OH)CH₂- or -CH₂CH₂CH(CH₃)-];
R² represents a hydroxyl group or an acyloxy group selected from formyloxy and acetoxy;
R³ represents a group selected from 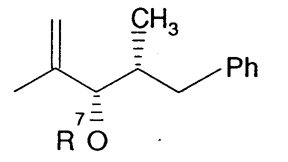 (where R⁷is a hydrogen atom or an acetyl group), (CH₃) 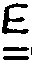 CHCH(CH₂R⁸)CH₂Ph (where R⁸ is a hydrogen or a hydroxyl group),-C(CH₂OH) 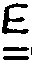 CHCH(CH₃)CH₂Ph, -C(=CH₂)CH(OH)CH (CH₂OH)CH₂Ph,-C(=CH₂)CH(NHCOCH₃)CH(CH₃)CH₂Ph, -C(CH₂NHCOCH₃) 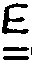 CHCH(CH₃)CH₂Ph and 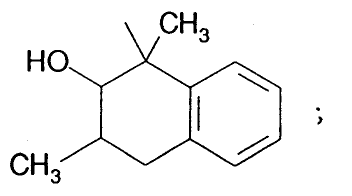 R⁴,R⁵ and R⁶ may each independently represent a hydrogen atom or a methyl group; and salts thereof.

2. Compounds according to Claim 1 in which R⁴, R⁵ and R⁶ represent hydrogen atoms.

3. Compounds according to Claim 1 or Claim 2 in which R¹ represents a group

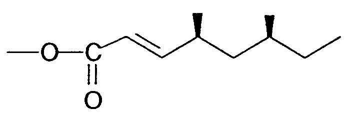

4. Compounds according to any preceding claim in which R² represents a hydroxyl group.

5. Compounds according to any preceding claim in which R³ represents a group 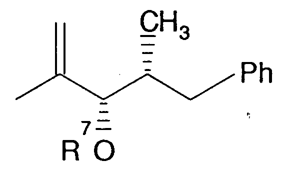 where R⁷ is a hydrogen atom or an acetyl group.

6. [1S-[1α(4R*,5S*),3α,4β,5α,6α(2E,4R*,6R*),7β]] 1-(4-Acetyloxy-5-methyl -3-methylene -6-phenylhexyl)-6,7-dihydroxy-2,8-dioxabicyclo[3.2.1]octane-3,4,5-tricarboxylic acid, 6-(4,6-dimethyl-2-octenoate) and physiologically acceptable salts thereof.

7. A compound according to any preceding claim for use in therapy.

8. A compound according to any of Claims 1 to 6 for use in the treatment of conditions where a lowering of the level of blood plasma cholesterol in animals, including humans, would be beneficial.

9. A compound according to any of Claims 1 to 6 for use in the treatment of fungal infections in a human or non-human animal patient.

10. A method of treatment of the human or non-human animal body to combat diseases associated with hypercholesterolemia and/or hyperlipoproteinemia or to combat fungal diseases, which method comprises administering to said body an effective amount of a compound as claimed in any of Claims 1 to 6 which inhibits squalene synthase.

11. A pharmaceutical composition comprising a compound according to any of Claims 1 to 6 together with one or more carriers and/or excipients.

12. A pharmaceutical composition comprising an active amount of a compound as claimed in any of Claims 1 to 6 for use in the treatment of conditions where a lowering of the level of blood plasma cholesterol in animals, including humans, would be beneficial.

13. A pharmaceutical composition comprising an active amount of a compound as claimed in any of Claims 1 to 6 for use in the treatment of fungal infections in a human or non-human animal patient.

14. A pharmaceutical composition according to any one of Claims 11 to 13 in a form suitable for oral, buccal, topical, parenteral, implant, rectal, ophthalmic or genito-urinary administration or in a form suitable for administration by inhalation or insufflation.

15. A pharmaceutical composition according to any one of Claims 11 to 14 in unit dosage form.

16. Use of a compound according to any of Claims 1 to 6 in the manufacture of a medicament for the treatment of hypercholesterolemia and/or hyperlipoproteinemia in a human or non-human animal patient.

17. Use of a compound according to any of Claims 1 to 6 in the manufacture of a medicament for the treatment of fungal infections in a human or non-human animal patient.

18. A process for the preparation of a compound as claimed in Claim 1 which comprises:
(A) (in the preparation of compounds of formula (I) in which R² represents a hydroxyl group or an acetoxy group) reacting a compound of formula (II) 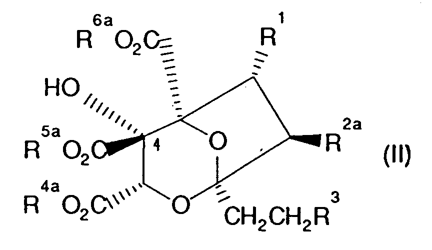 (wherein R¹ and R³ are as defined in Claim 1, R²^{a} is an acetoxy group and R⁴^{a}, R⁵^{a} and R⁶^{a} are protecting groups) to replace the 4-hydroxyl group with a hydrogen atom, followed by removal of the protecting groups present; or
(B) converting a compound of formula (I) to a different compound of formula(I).

19. Compounds of formula (III).

20. Compounds according to any of Claims 1 to 6 substantially as herein described.

21. Compositions according to any one of Claims 11 to 15 substantially as herein described.
